(19) Europäisches Patentamt
European Patent Office
Office européen des brevets

(11) Veröffentlichungsnummer : **0 413 792 B1**

(12) # EUROPÄISCHE PATENTSCHRIFT

(45) Veröffentlichungstag der Patentschrift :
**15.07.92 Patentblatt 92/29**

(51) Int. Cl.$^5$ : **A61K 37/02,** A61K 33/00

(21) Anmeldenummer : **90903818.4**

(22) Anmeldetag : **27.02.90**

(86) Internationale Anmeldenummer :
**PCT/EP90/00324**

(87) Internationale Veröffentlichungsnummer :
**WO 90/10455 20.09.90 Gazette 90/22**

(54) **ERZEUGNISSE, ENTHALTEND EIN LITHIUMSALZ UND EINEN TUMOR-NEKROSE-FAKTOR.**

(30) Priorität : **07.03.89 DE 3907244**

(43) Veröffentlichungstag der Anmeldung :
**27.02.91 Patentblatt 91/09**

(45) Bekanntmachung des Hinweises auf die Patenterteilung :
**15.07.92 Patentblatt 92/29**

(84) Benannte Vertragsstaaten :
**AT BE CH DE FR GB IT LI NL**

(56) Entgegenhaltungen :
**WO-A-87/03489**
**Proc. Natl. Acad. Sci. USA, vol. 86, December 1989, (Washington, US), R. Beyaert et al.: "Lithium chloride potentiates tumornecrosis factor-mediated cytotoxicity in vitro and in vivo", pages 9494-9498**
**Proceedings of AACR, vol. 29, March 1988, R. Knowles et al.: "Lithium chloride stimulates human monocytes to secrete tumornecrosis factor", page 433, abstract 1724**

(56) Entgegenhaltungen :
**Cancer, vol. 48, no. 12, December 1981, American Cancer Society, (Amsterdam, NL), R.S. Stein et al.: "Lithium carbonate andgranulocyte production: dose optimization", pages 2696-2701**

(73) Patentinhaber : **KNOLL AG**
**Knollstrasse 32**
**W-6700 Ludwigshafen (DE)**

(72) Erfinder : **KEMPENI, Joachim**
**Altdorfer Strasse 14 a**
**W-6730 Neustadt-Duttweiler (DE)**
Erfinder : **KLUGE, Michael**
**Am Huebaum 3**
**W-6701 Kallstadt (DE)**
Erfinder : **FIERS, Walter**
**Beukendreef 3**
**B-9120 Destelbergen (BE)**

(74) Vertreter : **Karau, Wolfgang, Dr. et al**
**BASF Aktiengesellschaft Carl-Bosch-Strasse 38**
**W-6700 Ludwigshafen (DE)**

EP 0 413 792 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Erzeugnisse, enthaltend ein Lithiumsalz und einen Tumor-Nekrose-Faktor.

Es ist bereits bekannt, daß man mit Tumor-Nekrose-Faktor α (TNF-α) oder Tumor-Nekrose-Faktor-β (TNF-β, auch als Lymphotoxin bezeichnet) sowie mit Muteinen dieser beiden Substanzen das Wachstum von Krebszellen hemmen kann, vgl. Eur. J. Biochem. 152, 515, 1985; EP 187 991, WO 86/04 606, EP 100 641.

Es wurde nun gefunden, daß man die Wirkung der Tumor-Nekrose-Faktoren durch Gabe von Lithiumsalzen steigern kann.

Gegenstand der Erfindung sind Erzeugnisse, enthaltend ein Lithiumsalz und einen Tumor-Nekrose-Faktor als Kombinationspräparat zur gleichzeitigen oder zeitlich abgestuften Anwendung bei der Behandlung von malignen Tumoren des Menschen und deren Begleiterscheinungen.

Unter der Bezeichnung maligne Tumoren sind Tumoren der blutbildenden Organe, insbesondere Leukämien und Lymphome sowie bösartige Tumoren verschiedener Organe zu verstehen. Zu diesen bösartigen Tumoren sind zu zählen insbesondere Tumoren der Lunge, des Gastrointestinaltraktes, des Urogenitaltraktes, der Brust sowie der Haut und ihren Anhangsgebilden.

Zu den Begleiterkrankungen bei malignen Tumoren zählen insbesondere maligne Ergüsse in Körperhöhlen im Gefolge dieser Tumoren.

Unter der Bezeichnung Tumor-Nekrose-Faktor sind TNF-α, TNF-β (Lymphotoxin) sowie deren wirksame Muteine zu verstehen.

Der Tumor-Nekrose-Faktor wird in der Regel in einer täglichen Menge von 1 bis 500 μg/m² Körperoberfläche, insbesondere in einer täglichen Menge von 40 bis 400 μg/m² angewendet. Die Applikation erfolgt dabei intravenös, subcutan, intraperitoneal oder intra- bzw. peritumoral.

Als Lithiumsalze kommen insbesondere das Carbonat, D,L-Hydrogenaspartat, Sulfat, Orotat, Atetat sowie das Chlorid in Betracht. Die Lithiumsalze werden oral, intravenös, intraperitoneal oder intra- bzw. peritumoral in einer solchen Menge appliziert, daß der Blutspiegel an $Li^+$-Ionen zwischen 0,4 und 1,8 mmol, insbesondere jedoch zwischen 0,7 und 1,4 mmol liegt.

Der Tumor-Nekrose-Faktor und das Lithiumsalz können in bekannten galenischen Applikationsformen angewendet werden, vgl. Rote Liste 1988, Nrs. 70223 bis 70227, EP-A-209 030.

Die Überlegenheit der neuen Kombinationstherapie konnte wie folgt in vitro und in vivo gezeigt werden:

### In vitro-Experimente

24 Stunden vor der Behandlung wurden die Zellen in einer Konzentration von 5 bis $10^3$ Zellen/Kolben in einem zellspezifischen Medium auf Mikrotiter-Platten ausgestrichen. Anschließend wurden mehrere TNF-Verdünnungen zugegeben. Zum Testen von LiCl wurde dies den Zellen 2 Stunden vor der Behandlung mit TNF in verschiedenen Konzentrationen zugesetzt. Nach einer Inkubationszeit von 72 Stunden bei 37°C wurden die adhärenten Zellen fixiert und 15 Minuten lang mit einer Lösung gefärbt, die 0,5 % (w/v) Kristallviolett, 4 % (v/v) Formaldehyd, 30 % (v/v) Ethanol und 0,17 % (w/v) NaCl enthielt. Die Kolben wurden gründlich mit Leitungswasser ausgespült, die adhärenten Zellen wurden dann in 33 % (v/v) Essigsäure (0,1 ml/Reagenzglas) gelöst. Der freigesetzte Farbstoff wurde mit Hilfe eines Immunoreader NJ-2000 (Nippon Intermed, Tokio, Japan) spektrophotometrisch gemessen.

Der Einfluß von LiCl auf die zytolytische/zytostatische Aktivität von TNF wurde an zwei Zellinien von Mäusen (L929 und WHI 164, Klon 13) und fünf Zellinien vom Menschen (MCF7-AZ, ME180, Bt20 und HeLa D98(AH2) untersucht. Sämtliche Zellinien waren gegen TNF empfindlich. LiCl verstärkte die zytotoxische Wirkung von TNF auf sämtliche Mäuse-Zellinien und drei der untersuchten Zellinien von Menschen in dosisabhängiger Weise (Fig. 1). In Gegenwart von LiCl erfolgte bei TNF-Konzentrationen, die allein praktisch keine Wirkung zeigten, eine fast vollständige Zellabtötung (Fig. 2).

### In vivo-Experimente

Die subkutane (s.c.) Injektion von $10^6$ L-929-Zellen bewirkte bei 6 Wochen alten weiblichen Nacktmäusen (nu/nu; IFA-CREDO, Brüssel, Belgien) ein schnelles Wachstum nicht-invasiver Tumoren. Die Tumoren wurden peritumoral (tägliche s.c.-Injektion in die Nähe des Tumorsitzes, jedoch außerhalb des Knotens) mt TNF und/oder LiCl über mehrere Perioden von 5 Tagen im Abstand von 2 Tagen behandelt. Die TNF-Dosen wurden wöchentlich gesteigert, während die LiCl-Dosis bei 1 mg/Maus konstant gehalten wurde. Im Vergleich zu TNF alleine war die Wirkung der Kombination von TNF und LiCl stark ausgeprägt (Fig. 3; LiCl alleine zeigte keine Wirkung). Ab der zweiten Woche nekrotisierten außerdem einige Tumore nach der Behandlung mit TNF oder

TNF/LiCl. Außer bei einer Maus (völliges Verschwinden des Tumors nach 2 Wochen Kombinationstherapie) war in beiden Fällen die Wachstumshemmung allerdings nicht vollständig. Bei längerer Kombinationstherapie (über mehrere Wochen) traten keine Nebenwirkungen auf. Die mit TNF behandelten Mäuse überlebten länger als die Kontrollmäuse; nach einer Kombinationstherapie überlebten die Tiere allerdings wesentlich länger (Fig. 4).

Darüber hinaus erhielten Nacktmäuse s.c.-Injektionen von HeLa D/98/AH2-Zellen ($3 \times 10^6$), die langsamer wachsende Tumore erzeugten. Es wurde dasselbe Protokoll angewandt wie oben beschrieben. Auch in diesem Falle zeigte TNF alleine einen geringen Effekt auf das Turmorwachstum. Eine Kombinationstherapie mit LiCl bewirkte hingegen eine Wachstumshemmung, während eine Nekrose nicht sichtbar war. Nach längerer Behandlung mit der Kombination waren zwei (von sechs) Mäusen völlig tumorfrei (Fig. 5).

Legenden zu den Figuren 1 bis 5

Fig. 1 Verstärkung der TNF-bedingten Zytotoxizität durch LiCl bei verschiedenen malignen Zellinien
Die Zellen wurden in einem in-vitro-Assay wie oben beschrieben getestet. Die prozentuale Potenzierung ist für eine steigende LiCl-Konzentration bei konstanter TNF-Konzentration dargestellt.

$$\text{Prozentuale Potenzierung} = \left[ 1 - \frac{\text{Abs (TNF + LiCl)}}{\text{Abs (TNF)}} \right] \times 100$$

(Abs entspricht dem Absorptionswert bei 590 nm für die fixierten und gefärbten Zellen nach der Behandlung mit den in Klammern stehenden Substanzen.)
LiCl alleine hatte keinerlei Wirkung auf die Lebensfähigkeit der Zellen. Aufgeführt sind die Werte eines repräsentativen Experimentes.

Fig. 2 Dosisabhängige Wirkung von LiCl auf die durch TNF hervorgerufene Zytotoxizität von L929-Zellen
Die Zellen wurden in dem oben beschriebenen in-vitro-Assay getestet. Die prozentuale Überlebensdauer wurde im Vergleich zu den steigenden TNF-Konzentrationen eingetragen. Für jede LiCl-Konzentration entspricht die prozentuale Überlebensdauer dem nach der Behandlung mit TNF + LiCl ermittelten Zell-Anfärbungswert, ausgedrückt als Prozentsatz des Zell-Anfärbungswertes, wie er in Kulturen ermittelt wurde, die mit LiCl, nicht aber mit TNF behandelt wurden (= 100 %-Wert). Die oben rechts eingefügte Figur zeigt den Einfluß von LiCl auf das Zellwachstum und -überleben in Abwesenheit von TNF.

Fig. 3 Wirkungen der Behandungen mit TNF und TNF + LiCl auf das Wachstum von s.c L-929-Tumoren bei nu/nu-Mäusen
Die Tumorgröße (Produkt der größten rechtwinkligen Durchmesser) ist im Vergleich zur Zeit (Tage nach der Injektion der Tumorzellen) eingetragen. Sämtliche Mäuse (sechs pro Gruppe) erhielten an den durch einen Pfeil markierten Tagen 0,1 ml einer Mischung beider Substanzen peritumoral injiziert. TNF wurde in folgenden Dosen verabreicht: 5 µg (1. Woche), 10 µg (2. und 3. Woche), 15 µg (4. Woche) und 20 µg (5. Woche); die Konzentration von LiCl betrug in sämtlichen Fällen 1 mg/Injektion.

Fig. 4 Überlebensdauer von Mäusen, denen L929-Zellen s.c. injiziert und die mit TNF und/oder LiCl behandelt wurden
Die Resultate gelten für die in der Legende zu Fig. 3 aufgeführten Mäuse.

Fig. 5 Wirkungen der Behandlung mit TNF und/oder LiCl auf das Wachstum von s.c HeLa D98/AH2-Tumoren bei nu/nu-Mäusen
Die Tumorgröße wurde nach Attia und Weiss (1966) ($0,4\ ab^3$) berechnet und ist als semilogarithmische Skala im Vergleich zur Zeit (Tage nach Injektion der Tumorzellen) eingezeichnet. Sämtliche Mäuse (6 pro Gruppe) erhielten 0,1 ml einer Lösung von TNF oder LiCl bzw. einer Mischung von TNF und LiCl an den durch einen Pfeil markierten Tagen peritumoral injiziert. TNF wurde in folgenden Dosen verabreicht: 10 µg (1. Woche), 15 µg (2. und 3. Woche) und 25 µg (4. und 5. Woche); die Konzentration von LiCl betrug in allen Fällen 1 mg/Injektion.

## Patentansprüche

1. Erzeugnisse, enthaltend ein Lithiumsalz und einen Tumor-Nekrose-Faktor als Kombinationspräparat zur gleichzeitigen oder zeitlich abgestuften Anwendung bei der Behandlung von malignen Tumorerkrankungen des Menschen und deren Begleiterscheinungen.

## Claims

1. Products containing a lithium salt and a tumor necrosis factor as combination product for simultaneous or sequential use for the treatment of malignant tumorous diseases in humans and their concomitant phenomena.

## Revendications

1. Produits contenant un sel de lithium et un facteur nécrose-tumeur (TNF) en tant que préparation de combinaison pour utilisation simultanée ou échelonnée dans le temps lors du traitement de tumeurs malignes de l'homme et leurs phénomènes secondaires.

FIG.1

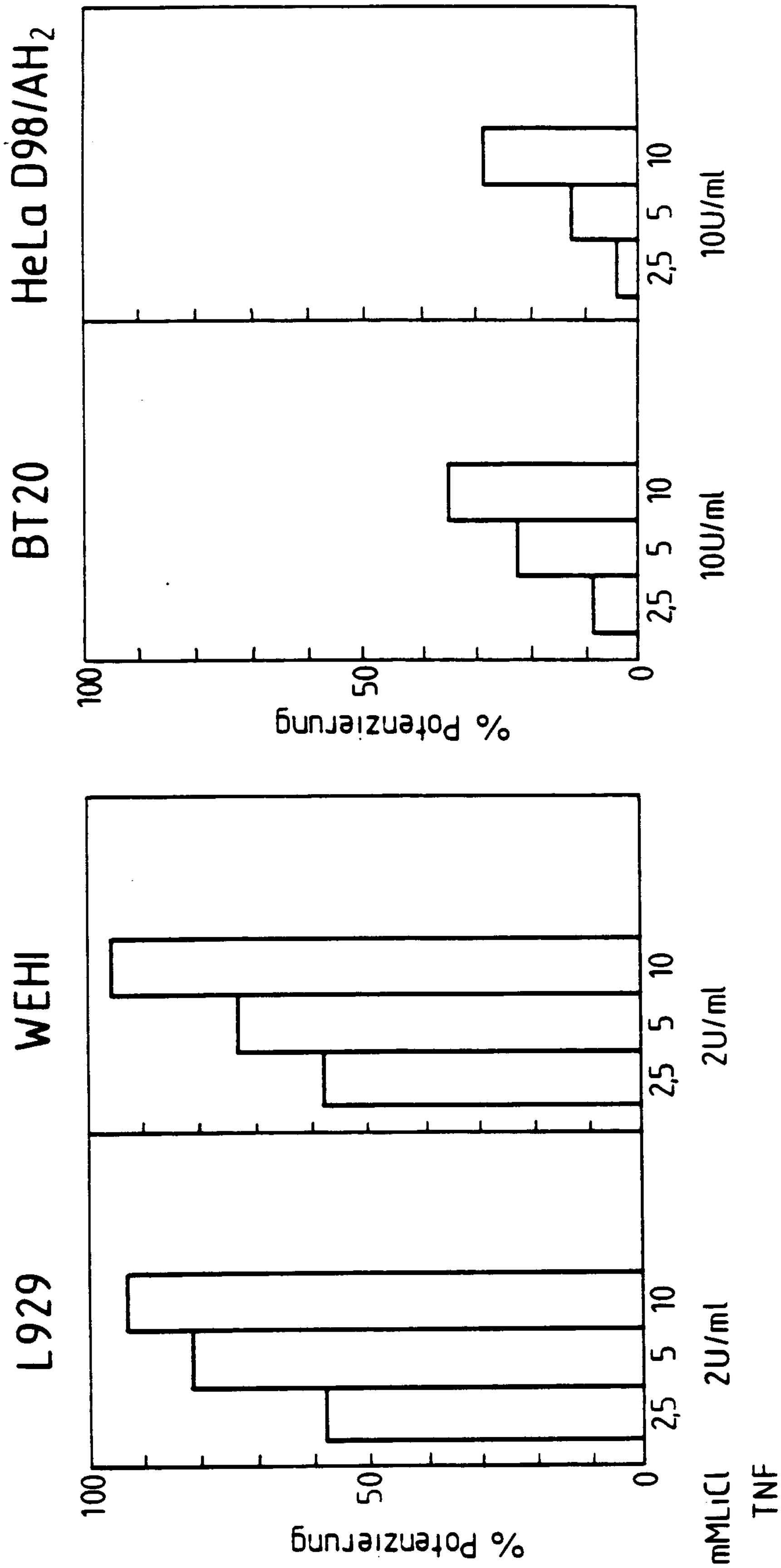
L929
WEHI
BT20
HeLa D98/AH2
% Potenzierung
100
50
0
2,5
5
10
2U/ml
10U/ml
mMLiCl
TNF

FIG. 2

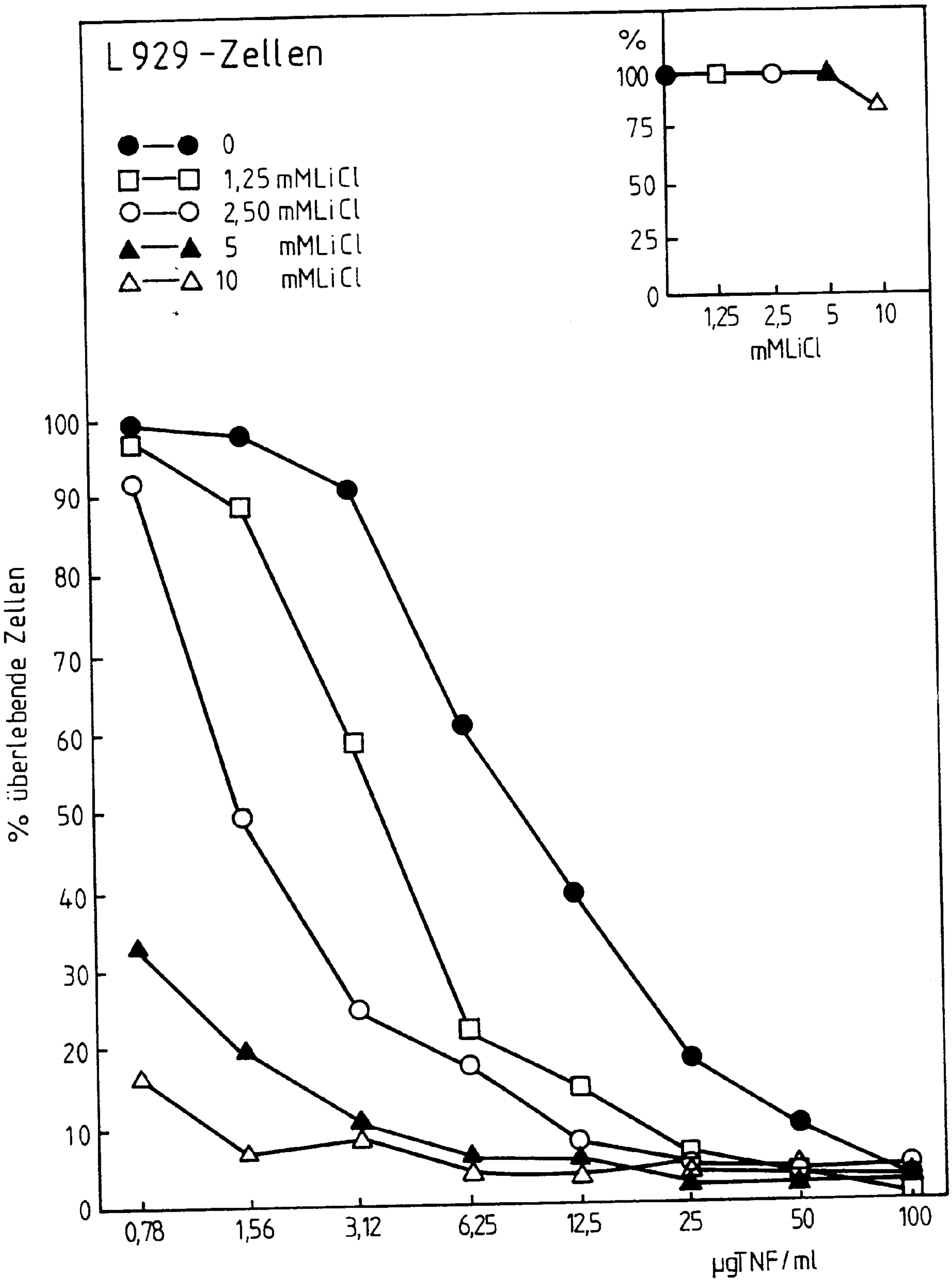
L 929 -Zellen
0
1,25 mMLiCl
2,50 mMLiCl
5 mMLiCl
10 mMLiCl
%
100
75
50
25
0
1,25
2,5
5
10
mMLiCl
% überlebende Zellen
100
90
80
70
60
50
40
30
20
10
0
0,78
1,56
3,12
6,25
12,5
25
50
100
µgTNF/ml

FIG.3

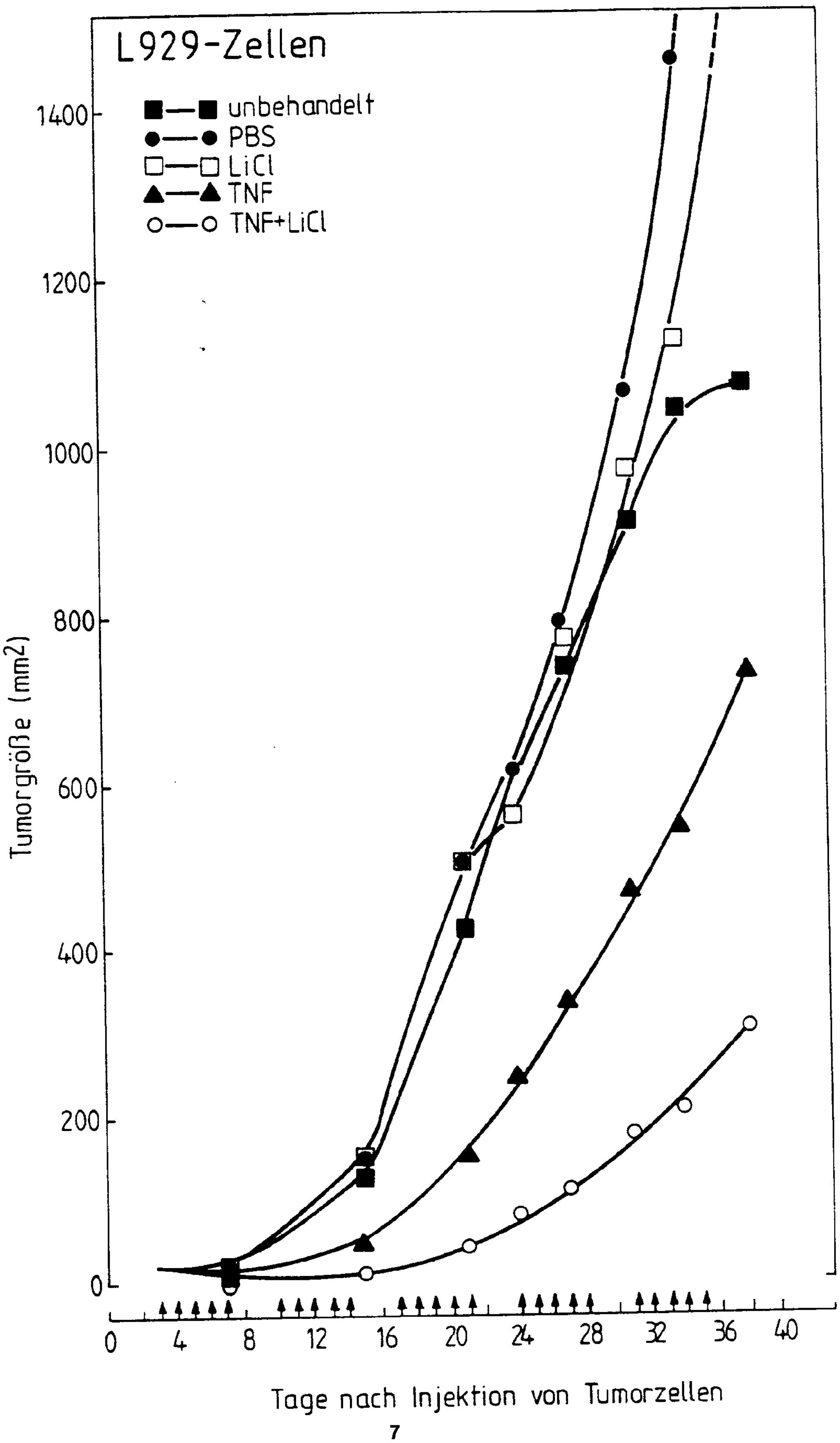
L929-Zellen
unbehandelt
PBS
LiCl
TNF
TNF+LiCl
1400
1200
1000
800
600
400
200
0
Tumorgröße (mm2)
0
4
8
12
16
20
24
28
32
36
40
Tage nach Injektion von Tumorzellen

FIG. 4

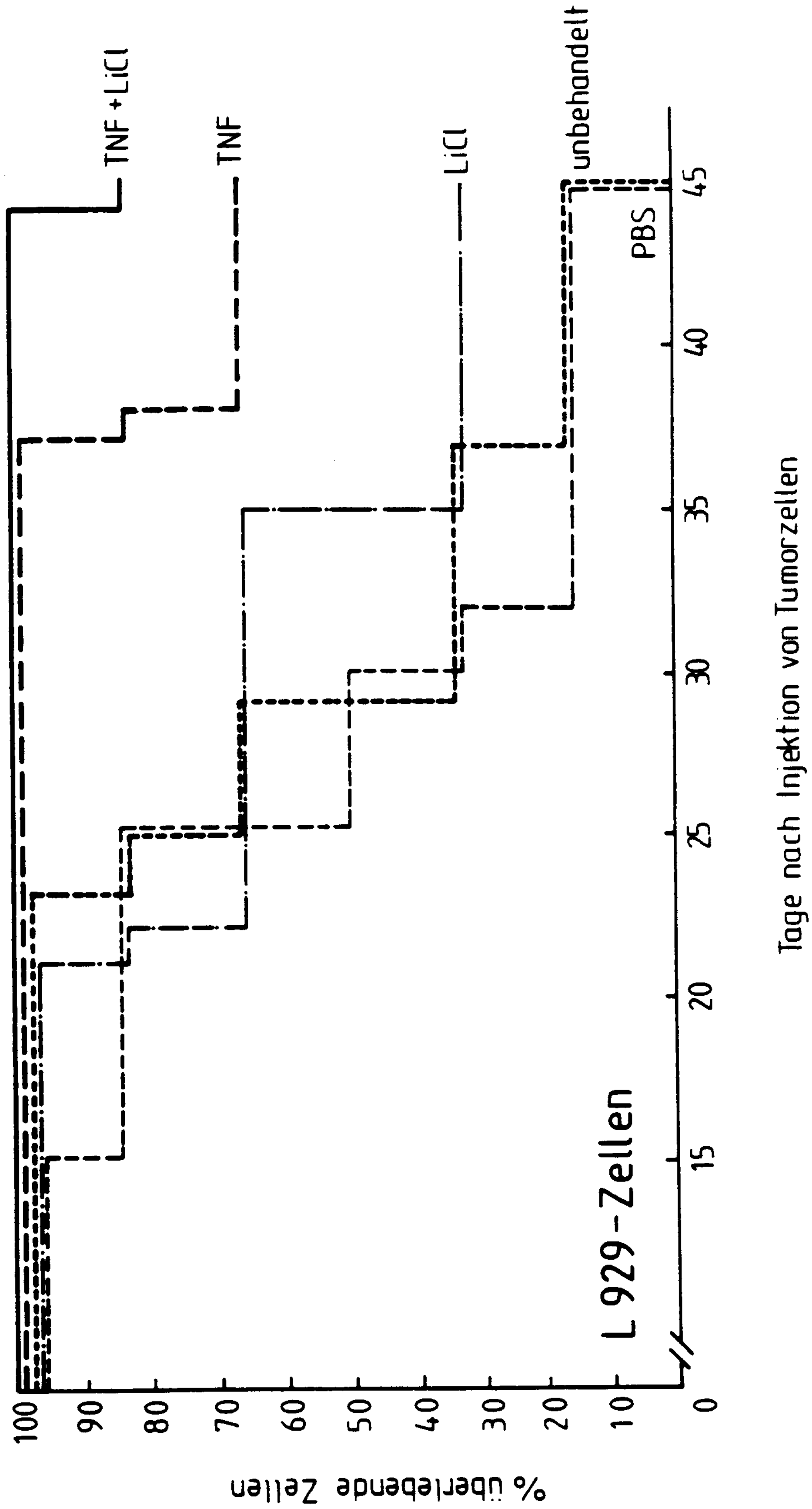
L 929-Zellen
TNF + LiCl
TNF
LiCl
unbehandelt
PBS
% überlebende Zellen
100
90
80
70
60
50
40
30
20
10
0
15
20
25
30
35
40
45
Tage nach Injektion von Tumorzellen

FIG.5

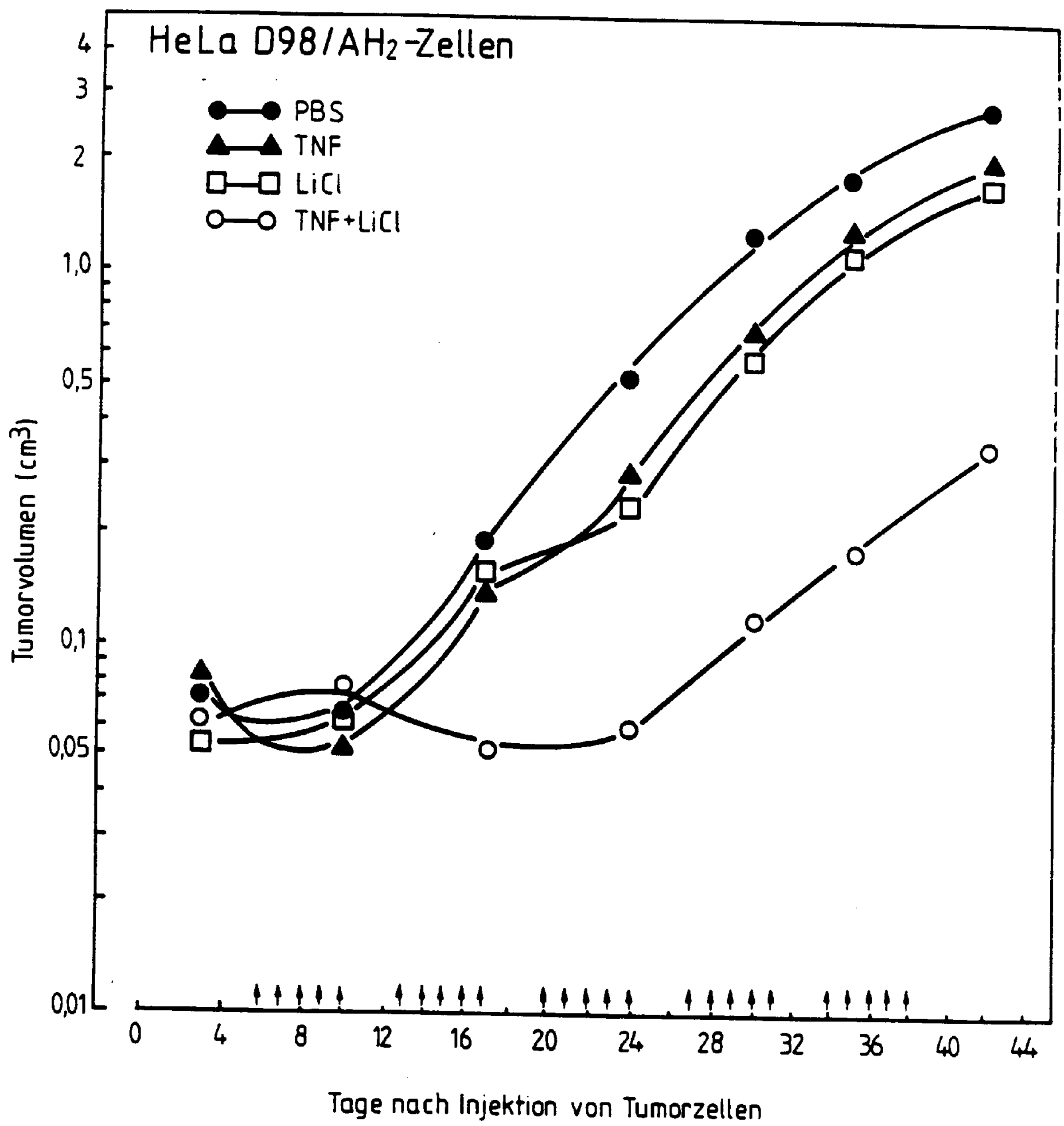
HeLa D98/AH2-Zellen
PBS
TNF
LiCl
TNF+LiCl
Tumorvolumen (cm3)
4
3
2
1,0
0,5
0,1
0,05
0,01
0
4
8
12
16
20
24
28
32
36
40
44
Tage nach Injektion von Tumorzellen